(19) **Europäisches Patentamt**

**European Patent Office**

**Office européen des brevets**

(11) Publication number : **0 406 303 B1**

(12) **EUROPEAN PATENT SPECIFICATION**

(45) Date of publication of patent specification :
16.08.95 Bulletin 95/33

(51) Int. Cl.$^6$ : **G01N 33/535,** G01N 33/569, G01N 33/577, C12Q 1/28

(21) Application number : **89904313.7**

(22) Date of filing : **15.03.89**

(86) International application number :
**PCT/US89/01060**

(87) International publication number :
**WO 89/08844 21.09.89 Gazette 89/23**

(54) **ASSAY FOR HUMAN IMMUNODEFICIENCY VIRUS.**

(30) Priority : **15.03.88 US 168493**

(43) Date of publication of application :
**09.01.91 Bulletin 91/02**

(45) Publication of the grant of the patent :
**16.08.95 Bulletin 95/33**

(84) Designated Contracting States :
**AT BE CH DE FR GB IT LI LU NL SE**

(56) References cited :
**EP-A- 0 196 873
EP-A- 0 248 534
WO-A-85/04903
JOURNAL OF VIROLOGY, vol. 62, no. 10, October 1988; B. CHESEBRO et al., pp. 3779-3788
CELL, vol. 47, no. 3, 07 November 1986; P.J. MADDON et al., pp. 333-348
EUROPEAN JOURNAL OF IMMUNOLOGY, vol. 16, 1986; pp. 1465-1468**

(56) References cited :
**JOURNAL OF CLINICAL MICROBIOLOGY, vol. 25, no. 7, July 1987, american Society for Microbiology, Washington, DC (US); T. MAT-SIU et al., pp. 1305-1307
VIROLOGY, vol. 141, no. 1, February 1985; M. SITBON et al., pp. 110-118
PROCEEDINGS OF THE NATL. ACADEMY OF SCIENCES USA, vol. 84, no. 10, May 1987; C. CHENG-MAYER et al., pp. 3526-3530**

(73) Proprietor : **THE UNITED STATES OF AMERICA as represented by the Secretary UNITED STATES DEPARTMENT OF COMMERCE
Washington, DC 20002 (US)**

(72) Inventor : **CHESEBRO, Bruce
779 Willow Creek Road
Corvallis, MT 59828 (US)**
Inventor : **WEHRLY, Kathy
512 Bailey Avenue
Hamilton, MT 59840 (US)**

(74) Representative : **Jump, Timothy John Simon et al
Venner Shipley & Co.
20 Little Britain
London EC1A 7DH (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method for AIDS diagnosis and monitoring of anti-AIDS drug therapy, and more particularly to a method of assaying for human immunodeficiency virus.

AIDS is a relatively newly recognized disease. To date, thousands of cases have been reported, and the disease has elicited great concern because of the rapid expansion of the epidemic and the high mortality rate of the disease. Its overwhelming prevalence among homosexual men with multiple sexual partners, users of illegal intravenous drugs, hemophiliacs, blood transfusion recipients, and close heterosexual contacts of members of the above high-risk groups strongly suggests that the disease spreads by the transmission of an infectious agent. The primary targets of affliction in the human body are specific subpopulations of T-cells. The severe immune deficiency of patients with AIDS results from an unusually low proportion of helper T-cells (T4 cells) in their lymphocyte population, thus reducing the availability of many T4 helper functions, among which is the production of antibodies by B-cells.

Retrovirus infection is known to lead to depressed immune function in animal systems. Analogizing the human response to these animal systems, a human retrovirus with a tropism for T-cells was considered a candidate in the etiology of human AIDS.

Several members of a family of human T-lymphotropic retroviruses (HTLV) have been isolated. One of these isolates was obtained from a patient with an aggressive form of T-cell lymphoma. This virus, designated as HTLV-I, has been etiologically linked to the pathogenesis of adult T-cell leukemia/lymphoma (ATLL). In vitro infection with HTLV-I can alter T-cell function and, in some cases, lead to T-cell death. Another member of the HTLV family was isolated from a patient with a T-cell variant of hairy cell leukemia, and was designated HTLV-II. Isolation of the HTLV-I and HTLV-II have been reported from cultured T-cells of patients with AIDS. Isolation of another retrovirus was reported from a homosexual patient with chronic generalized lymphadenopathy, a syndrome that often precedes AIDS and therefore is referred to as "pre-AIDS". Proviral DNA of HTLV-I was detected in the cellular DNA of two AIDS patients, and sera of some patients were shown to react with antigens of HTLV-I. The correlation between AIDS and serum antibodies to HTLV-I protein is weak.

It has now been shown that the primary cause of AIDS is a human T-lymphocytic retrovirus variant with limited cross-reactivities with the known HTLV subgroups. These new variants are designated HTLV-III, or, more recently, HIV.

Since AIDS and human immunodeficiency virus (HIV) are known to be transmitted by blood products, a method for detecting contamination of blood by HIV is needed to guarantee the safety of recipients of blood. Such a test method should be as sensitive and specific as possible, and should also be easily performed in a routine environment. Tests for viral markers such as reverse transcriptase, viral antigens, or nucleic acid sequences in fresh or cultured blood cells are slow and are presently not suitable for large-scale screening. On the other hand, serological tests for viral antibodies or antigens are well suited for this purpose, since HIV infected individuals are generally seropositive for antibodies to one or more HIV proteins.

Quantitative measurement of HIV, the agent of acquired immune deficiency syndrome, is currently a technically difficult procedure. Primary isolation of HIV from patients is usually done in PHA- or allo-genically-stimulated human lymphocyte blast cell cultures. This procedure was subsequently adapted using end-point dilution to give a quantitative (ID50) estimate of infectious virus. However, for many studies, an enumeration, or "plaque", assay would be highly desirable.

A plaque test has been described in HTLV-I-carrying cell lines by Harada et al., in J. Clin. Microbiol. 25, 1305 (1987). This assay is quantitative and useful for the LAV/HTLV IIIB strain of HIV, but it is less sensitive than the use of normal human lymphocyte blast cultures and does not work on wild-type HIV isolates. Furthermore, viruses recovered from such cells have the potential to be altered by recombination with HTLV-I-derived gene sequences, thus obscuring the true genetic structure of the original input HIV strain.

Previous work with other retroviruses has indicated that assay of virus-infected cells by infectious center assay on susceptible monolayers of cells adherent to plastic dishes is a highly sensitive quantitative detection method. Furthermore, focal areas of virus infection can be identified using virus-specific monoclonal or polyclonal antibodies, thus eliminating the requirement for a focal cytopathic effect at each site of infection, which can be difficult or impossible to obtain in certain virus-cell combination. Finally, if detection is done in living cells, biologically cloned viruses can be directly obtained from heterogeneous populations. Such an approach has not been possible with HIV because of the lack of suitable plastic-adherent target cells.

Gallo et al., in U.S. Patent 4,520,113, disclose a method for detecting antibodies to AIDS in sera by either a strip radioimmunoassay based on the Western Blot technique, or ELISA, an enzyme-linked immunosorbent assay.

Focal immuno assay (FIA) is a known assay technique, but it has not been used to assay for HIV-infected cells or HIV in the absence of cells because of the lack of appropriate target cells, specific anti-HIV antibodies, and the requisite procedural details to make the test work for HIV.

The use of a FIA on live cells to quantize C-type murine leukemia viruses (MuLV) and dual-tropic recombinant mink cell focus-inducing (MCF) viruses was described by M. Sitbon et. al., in Virology 141, 110-118 (1985). In the described assay, tissue culture dishes were firstly seeded with mink or mouse SC-1 cells and a sample of cell-free virus was then added thereto. The cells were then incubated, exposed to a first antibody reactive with virus protein and then to a second antibody, reactive with the first antibody, which is conjugated to a marker. The number of marker-positive foci was then counted, in order to determine the quantity of virus present in the sample.

Both T.C. Chanh et. al., Eur. J. Immunol. 1986. 16:1465-1468, and EP-A-0248534 disclose the production of monoclonal antibodies to HIV (HTLV-III). In the work reported in the former reference, the monoclonal antibodies were raised using a chemically synthesized peptide, corresponding to the gp 160 protein of HIV, and the latter reference describes the antibodies as raised, inter alia, to viral lysates derived from the growth of HTLV-III in a human lymphoma (H9) cell line.

In "The T4 Gene encodes the AIDS virus receptor and is expressed in the Immune system and the brain" (Cell. Vol. 47, 333-348 1986) P.J. Maddon et. al. reported, inter alia, that a HeLa cell line, transformed with functional T4 cDNA, can be infected with the AIDS virus.

Most cell lines used for laboratory HIV infection studies grow as suspension cultures in liquid medium. In this case, rare infected cells float around in the cultures, spreading infection randomly. This can only be detected when the level of infected cells has spread enough to be detected by immunofluorescence or reverse transcriptase analysis. This usually takes from about two to four weeks for low levels of initial infection.

It is an object of the present invention to overcome deficiencies in the prior art, such as those noted above, and to provide an assay for HIV-infected cells, or a test for cell-free HIV. The invention, therefore, can be used as a test for diagnosing AIDS, or for monitoring levels of HIV or HIV-infected cells in samples obtained from humans, or produced in laboratory situations.

According to a first aspect of the invention, there is provided a method for assaying for human immunodeficiency virus, comprising seeding dishes with cells susceptible to infection and capable of growing on the dishes, adding a sample to be tested to the dishes, incubating the cells, exposing the cells to a first antibody and to a marker-conjugated antibody, reactive with the first antibody and counting the number of foci of marker-positive cells per dish, characterised in that the seeded cells comprise a human carcinoma cell line, into which a CD4 gene encoding the HIV receptor molecule T4 has been transferred, and are sensitive to infection by wild-type HIV, the first antibody is reactive with a HIV protein and the number of foci, of marker-positive cells, is counted in order to determine the number of HIV infected cells in the dish.

In a second aspect, the invention provides a method for detecting HIV-infected cells in a sample, comprising seeding dishes with first cells susceptible to infection, seeding the dishes with second cells from the sample, incubating the cells, exposing the cells to a first antibody, exposing the cells to a marker-conjugated antibody, reactive with the first antibody, and counting the number of foci of marker-positive cells, characterised in that the first cells comprise a human carcinoma cell line, into which a CD4 gene encoding the HIV receptor molecule T4 has been trasnferred, and are susceptible to wild-type HIV infection, the first antibody is reactive with HIV proteins, and the number of foci of marker-positive cells is counted, to determine the number of HIV infected cells in the sample.

According to a third aspect, the invention provides Target cells for use in focal immunoassays for HIV, comprising a human carcinoma cell line into which a CD4 gene encoding the HIV receptor molecule T4 has been transferred.

Further aspects and preferred features of the invention are set out in claims 2-11, 13-15 and 17-20 appended hereto.

Thus, the assay according to the present invention is a focal immuno assay (FIA) which uses HIV-specific antibodies and indirect immunological assays such as immunocyto-chemistry, autoradiography, or immuno-fluorescence, to detect local areas, or foci, of HIV infection in susceptible target cells growing in monolayers.

The test according to the present invention is both quantitative and highly sensitive. On laboratory HIV strains a single HIV-infected cell can be detected in a sample containing one million uninfected cells, and higher percentages of infected cells can be accurately quantitated. This procedure allows monitoring of the effects of drug therapy on levels of HIV expression in AIDS patients, as well as providing a quantitative measure of HIV expression in asymptomatic HIV antibody-positive individuals. The test according to the present invention is rapid, requiring only two to four days to complete, which is a major advantage compared to presently available tests.

The test according to the present invention is more sensitive for HIV-infected cells than for cell-free HIV. Nevertheless, the test does work with cell-free virus, and can be used to measure virus neutralizing antibody more quantitatively, since individual foci of virus infection can be counted.

To test the efficiency of the present invention, various dilutions of HIV or HIV-infected cells were placed into dishes previously seeded with cells susceptible to HIV infection. After short-term culturing, the cells were exposed to antibody reactive with HIV proteins or a monoclonal antibody for the gp 120 envelope protein of the LAV/HTLV IIIB strain of HIV, exposed to marker-conjugated antibody reactive with the first antibody used, and the dishes were then examined by appropriate means such as a fluoroescence microscope or a light-microscope, and the number of foci of marker-positive cells per dish were counted.

A process according to the present invention to test for the presence and/or extent of HIV infection involves the steps set forth above, except for addition of HIV or HIV- infected cells.

To obtain the appropriate target cells, HeLa cells were infected with a retroviral vector containing the CD4 gene and neomycin resistance gene. After infection, the cells were grown in the presence of the neomycin analog, G418, and resistant clones were selected. Of eighteen clones analyzed, four were found to express cell surface CD4 detectable by membrane immuno-fluorescence using the monoclonal antibody OKT4, and one clone, HT4-6C, was used in subsequent studies, ATCC number CRL-9631.

BRIEF DESCRIPTION OF THE DRAWINGS

Figure 1 shows the immunoprecipitation of HIV proteins.
Figure 2 shows foci of a variety of HIV(LAV strain)-infected HT4-6C cells.
Figure 3 shows the foci of HIV-infection in HT4-6C cells.
Figure 4 shows the correlation of HIV-infected lymphoid cells detected by FIA and by indirect immuno-fluorescence at various times after infection.
Figure 5 shows FIA of HIV (LAV strain) from infected A3.01 cell cultures used to infect HT4-6C cell monolayers.

DETAILED DESCRIPTION OF THE INVENTION

The present invention uses a focal immunoassay (FIA) which uses HIV-specific antibodies and indirect immunoassay techniques to detect local areas of HIV infection in susceptible target cells growing in monolayers on plastic dishes.

Varying dilutions of the sample to be tested, whether unknown or known to contain HIV or HIV-infected cells, are placed into dishes seeded one day previously with cells susceptible to HIV infection. Any suitable growth medium may be used. Selection of cells susceptible to HIV infection is important, although not limited to any single type of cells. However, the susceptible target cell must be one which will grow attached to the testing dishes, preferably formed of plastic. Suitable target cells are mentioned in the examples below.

Two to four days later growth medium is removed, and cells may be fixed, for example by methanol or formaldehyde or other conventional suitable fixers for from about five to about ten minutes. After optional rinsing of the fixed cells, e.g. with conventional aqueous buffer, the cells are exposed to an appropriate dilution and volume of antibody reactive with HIV proteins.

Again, the selection of anti-HIV antibody is an important consideration, and it is necessary to select the anti-HIV antibody from among those which are capable of detecting HIV in fixed or live infected cells with a high degree of sensitivity. Suitable materials are mentioned in the examples.

After exposure to the antibody, for thirty minutes, the cells are washed. The so-treated cells are then exposed to marker-conjugated antibody reactive with the first antibody. The antibody may be conjugated with any suitable marker material such as fluorescein isothiocyanate (FITC) which may be purchased from commercial sources, although clearly other materials can be used.

After further incubation, e.g. for about thirty minutes, the dishes are again washed, e.g. with aqueous buffer. Next, the cells are either fixed, e.g. with methanol or 2% formalin or reacted with suitable chemicals to visualize conjugated antibodies. After optional further washing and removal of fluid, the dishes are subjected to examination, such as in a fluorescence or light microscope, depending upon the type of marker used.

It was necessary to develop a susceptible target cell for use in the assay of the present invention. A susceptible target cell was developed which would grow attached to plastic dishes. In order to accomplish this, the CD4 gene encoding the HIV receptor molecule, T4, was transferred to a human carcinoma cell line, HeLa. Eighteen different T4-positive HeLa cell clones were derived and screened for HIV infection. Many were negative, and among the positive clones different levels of HIV infectibility were seen. The best clones were chosen for further experiments.

The anti-HIV antibody was selected as follows. Initially, several mouse monoclonal antibodies specific for the HIV envelope protein, gp120, were generated, which were capable of detecting some HIV strains in methanol-fixed or live infected cells with high sensitivity. These monoclonal antibodies were used in the initial development of procedural details of the FIA and selection of the best target cell clone. However, because it was feared that these antibodies might not detect wild type HIV strains as well as human AIDS patient serum, human AIDS patient serum was also used for HIV detection in the FIA. Initially, this was a problem, since AIDS patient serum contains many antibodies other than anti-HIV. However, by extensive absorption of serum or by selection of patients with low background, non-specific reactivity could be eliminated and such sera were effective in the test.

Monoclonal antibodies reactive with the envelope protein of HIV were generated. These antibodies immunoprecipitated envelope protein from HIV-infected cells or virions, and were shown to be specific for gp 120 by their reactivity with cells infected with Vaccinia virus encoding HIV gp 120.

The second step in the detection method according to the present invention uses marker-conjugated antibodies reactive with the human or mouse immunoglobulins used in the first step. These marker-conjugated antibodies can be purchased from commercial sources, such as Cappel Laboratories, in West Chester, PA.

After another incubation of about thirty minutes, the dishes were washed twice again with buffer and fixed again with methanol or 2% formalin or processed to develop marker-specific staining. After one additional aqueous wash, fluid was removed, and the dishes were examined in a suitable means, such as a fluoroescence microscope or light microscope or by autoradiography.

The number of foci of marker-positive cells per dish were then counted. Usually these foci consist of from about 1 to about 30 cells in a small area sometimes containing one or more cells with multiple nuclei (about 2 to more than 30). Since from about one to about five million lymphocytes or monocytes can be placed in a single dish, and one can detect a single focus in a dish, the assay can detect approximately one infected cell per one to five million cells tested.

Other variables which have been dealt with to make the assay of the present invention successful include the density of the target seeding ($5 \times 10^4$ per 35 mm dish), number of days between infection and focus detection (2 to 4 days), type of fixation, and method of observation.

To perform the assay for HIV-infected cells, monolayer cultures of HT4-6C cells were used to detect A3.01 human T lymphocytes infected in vitro with the LAV strain of HIV. Cells from infected A3.01 cultures were placed into dishes seeded one day previously with HT4-6C cells. The following day, suspended cells were removed, and the medium was changed. Three days later, the foci of infected cells were identified by the focal immunofluorescence assay using monoclonal antibody 902, reactive with HIV envelope protein. Multiple foci of infected HT4-6C cells were seen. Immunofluorescent cells were often multinucleated, but also appeared grouped in clusters of 10-20 mononuclear cells, as shown in Figures 2A and 2B. The observation of clusters of HIV infected mononuclear cells suggested that the LAV strain of HIV might be able to spread in HT4-6C cells without cell-to-cell fusion.

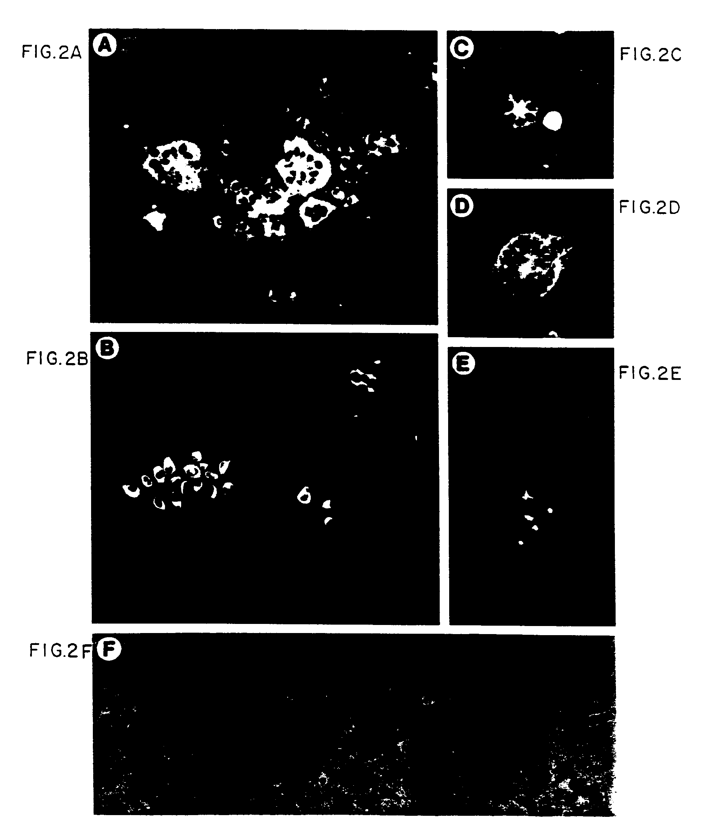

A similar assay was effected using three day PHA-stimulated human lymphocytes infected with a patient HIV isolate not previously adapted to growth in in vitro lymphoid cell lines. In this case, foci were detected using a polyclonal human anti-HIV AIDS patient serum, and the foci observed usually consisted of multinucleated giant cells, either alone or in groups of two or three together, as shown in Figure 2C.

Focal immunofluorescence assay was also successfully carried out using live infected HT4-6C cells without methanol fixation, as seen in Figure 2D. Under these conditions, foci were less obvious and few, if any, fluorescent multi-nucleated giant cells were seen. Examination of these cultures with visible light suggested that large syncytial cells were removed from the dishes during the multiple washing steps of the FIA procedure. The remaining fluorescent cells appeared to be mononuclear, and cells from each focus should potentially be a source of biologically cloned live HIV.

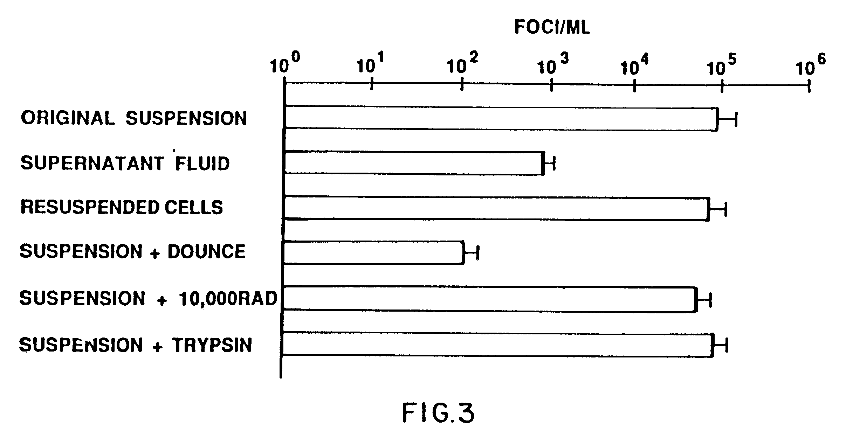

Since HIV-infected A3.01 lymphocyte suspension cultures contained both cell-free HIV virions and cell-associated HIV budding from live infected cells, it was of interest to determine which source of HIV was responsible for the foci detected by FIA in HT4-6C cells. FIA analysis of cell suspensions indicated that only 1% of the original foci were detectable after infection with cell-free supernatant fluid, as shown in Figure 3. Thus, 99% of the foci were due to cell-associated HIV, and these foci were mostly eliminated if the cells in suspension were killed by Dounce homogenization.

To test the sensitivity of the FIA in HT4-6C cells, this assay was compared with indirect immunofluoresence (IF) of HIV-infected A3.01 cells, or PHA blast cells for its ability to detect spread of HIV infection in lymphocyte suspension cultures. The immunofluorescence assay was not capable of scoring infected cells at an incidence below 0.1% because of a background of cells with non-specific fluorescence artifacts. However, the FIA could detect HIV infection at a level as low as 0.0001% cells (3 foci per $3 \times 10^6$ cells seeded in a 35 mm dish).

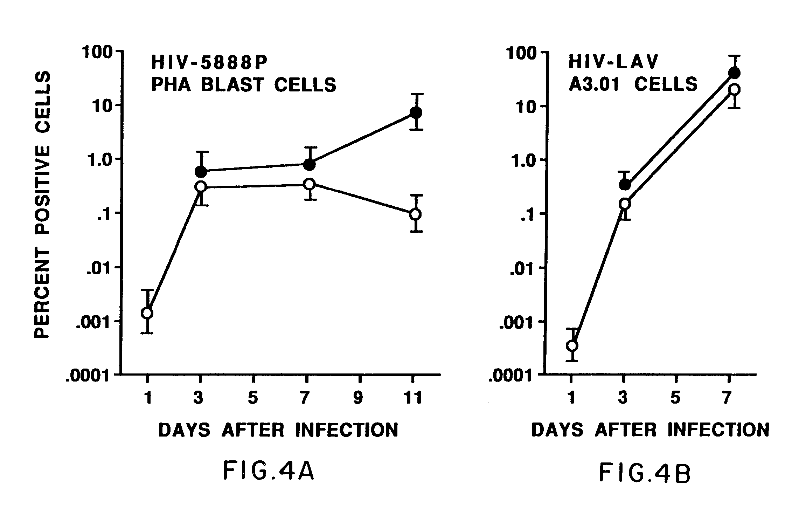

In a range where the two assays could be compared, there was an excellent correlation between the percent of cells detected in either assay, cf. Figures 4A and 4B, in which ● represents cells detected by IF assay and 0 represents cells detected by FIA. Thus, the FIA appeared to detect most or all of the cells expressing viral proteins. However, in older (11 day) PHA blast cell culture, the FIA and the immunofluorescence assays did diverge. At this time, the cultures appeared to accumulate dead or dying HIV-infected lymphocytes which were scored by immunofluorescence but not by FIA.

In these experiments, reverse transcriptase was also measured in the supernatant fluids of the infected A3.01 and PHA blast cell cultures. In both cases the FIA was found to be far more sensitive than the reverse transcriptase. In A3.01 cells RT became positive only after greater than 1% of cells were infected, whereas, in blast cells reverse transcriptase was positive when greater than 0.1% of cells was infected.

In other experiments with different HIV isolates, FIA and immunofluorescence assays were not always in agreement. In all cases, the FIA was more sensitive than either the reverse transcriptase or the immunofluorescence. However, the number of cells scored by FIA ranged from 5 to 100% of the cells scored by immunofluorescence. Also, in certain chronically infected cell lines, such as H9 cells infected with HTLV IIIB (ATCC # CRL8543), only 1-5% of the cells scored positive by FIA. These cells produced very low levels of cell-free infectious HIV.

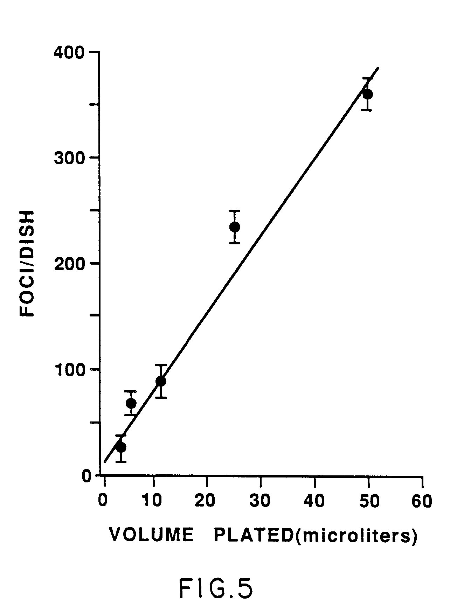

Initial experiments illustrated in Figure 3 suggested that the FIA in HT4-6C cells was also capable of detecting infection by cell-free HIV. Furthermore, a dilution titration of HIV on HT4-6C cells gave a one hit kinetic curve, i.e., a two-fold dilution gave a two-fold decrease in foci detected, as shown in Figure 5. Thus, each focus appeared to represent infection by a single HIV virion.

In order to test the sensitivity of this system, a comparison was made of titration of cell-free virions on HT4-6C, A3.01, and PHA blast cells. The results indicated that A3.01-adapted HIV, LAV strain, was detected with similar efficiency on HT4-6C, A3.01, and PHA blast cells. In the case of wild type HIV isolates not adapted to grow in A3.01 cells, HT4-6C cells detected only 0.1 to 1% of the infectivity observed in PHA blast cell cultures. These same virus isolates were not detected at all in A3.01 cell cultures.

Optimal conditions for detection of cell-free HIV appeared to be different for HIV(LAV) and wild-type HIV isolates. Polybrene and DEAE-dextran have frequently been used to increase the efficiency of retrovirus infection in vitro., cf. Toyoshima et al., Virology 38, 414 (1969). With HT4-6C cells, infection by the LAV strain of HIV was equally effective with or without DEAE-dextran; surprisingly, polybrene appeared to inhibit infection. In contrast, wild-type HIV isolates were detected only when cells were pretreated with DEAE-dextran; no treatment and infection in the presence of polybrene were not successful.

The cause of the relative inefficiency of initial infection of HT4-6C cells by wild-type HIV virions is unknown. This may be due to an insufficient quantity of T4 receptor molecules expressed on these cells. In this regard, 18 other HeLa clones infected with the CD4 expression vector were tested, and a large variation in sensitivity of HIV detection was found. Alternatively, it is also possible that other receptor molecules besides T4, and even membrane lipid composition, might contribute to more efficient HIV infection.

Because of its high potential sensitivity in detecting HIV-infected cells, the focal immunoassay of the present invention was used to detect HIV infection in lymphocytes obtained from AIDS patients. Patient cells were also co-cultivated with normal PHA blast cells, and culture fluids and cells were assayed for HIV at various times by FIA or by reverse transcriptase, cf. Table 1. Table 1. Use of a focal immunofluorescence assay and reverse transcriptase (RT) assay to detect HIV infection in AIDS patient lymphocytes co-cultivated with normal lymphocytes.

| Patient | Day 0 | 4 | 7 | 10 | 14 | 18 | 21 | 24 |
|---|---|---|---|---|---|---|---|---|
| | | | | | Foci/$10^6$ cells | | | |
| 6853 | <1 | 1920 | 610 | 140 | 35 | nt | nt | nt |
| 6588 | <1 | 21 | 1400 | 2800 | 700 | 420 | 280 | 1400 |
| 6585 | <1 | 70 | 1050 | 350 | 14 | 7 | 14 | 21 |
| 6435 | <1 | | <20 | 35 | 1400 | 700 | 140 | nt |
| 6598 | <1 | | <20 | 35 | 35 | 20 | <20 | <20 |
| 6597 | <1 | | <20 | 35 | <20 | 35 | <20 | 140 |
| 6428 | <1 | | | <20 | 840 | 560 | 350 | nt |
| 6426 | <1 | | | <20 | 513 | 280 | 280 | nt |
| 6589 | <1 | | | <20 | 70 | <20 | 35 | <20 |
| 6859 | <1 | | | | <20 | 63 | 70 | 175 |
| 6856 | <1 | | | | <20 | 35 | 30 | 23 |
| 6857 | <1 | | | | | <20 | 35 | <20 |
| 6848 | <1 | | | | | <20 | 35 | <20 |
| 6852 | <1 | | | | | | <20 | 23 |

[1] Threshold for positive by focal immunofluorescence assay was greater than 3 foci per 35mm dish of HT4-6C cells infected 4 days earlier with either 3 x $10^6$ freshly isolated Ficoll-Hypaque banded peripheral blood mononuclear cells, or in the case of cultured cells, 0.05 to 0.25 ml of lymphocyte culture suspension containing 0.25 to 1.25 X $10^5$ cells. Positive reverse transcriptase (RT) was greater than 200 cpm $^{32}$P-dTTP incorporated into DE81 paper binding material per 3.5 ul culture supernatant fluid in a 30 minute assay as described previously (23). Cultures positive for RT are indicated by underlining of the value shown for the FIA.

[2] Cultures of 8 patients in these experiments were negative by both FIA and RT at all times tested through 24 days.

[3] nt = not tested; blank values were negative by FIA (<20 foci/$10^6$ cells) and by RT.

[4] Cultures consisted of upright 25 cm$^2$ flasks with 10 ml RPMI 1640 with 10% fetal calf serum, 10 μg, PHA/ml, and 5 X 10-5 M, 2-mercaptoethanol plus 4 to 5 X $10^6$ Ficoll-Hypaque-banded peripheral blood mononuclear cells from AIDS patients and 4 to 5 X $10^6$ similar mononuclear cells from normal human blood stimulated in vitro 3 days earlier by culture at 2 X $10^6$ cells/ml with PHA at 10 μg/ml in medium as above. Cultures were fed every 2 to 3 days with medium containing 5% IL-2-positive culture medium (Electronucleonics, Rockville, MD), and every 7 days 4 X $10^6$ new 3 day PHA-stimulated normal human lymphocytes were added.

Analysis of these cultures by FIA was not difficult during the initial 14 to 18 days, however, after this time the cells in these cultures became quite toxic for the HT4-6C cells, and to avoid this effect fewer lymphocytes were added to each HT4-6C dish, thus reducing the sensitivity of the FIA.

[5] AIDS patients were randomly selected individuals from the NIAID 11th floor AIDS patient clinic at NIH, Bethesda, MD, who were positive for HIV antibodies by both ELISA and Western blot.

None of the patients' lymphocytes tested were positive when analyzed directly by FIA prior to in vitro cultivation. Therefore, based on the number of cells tested, these patient cell populations contained less than one FIA-positive cell per million cells. However, in many cases, co-cultivation in vitro for several days in the presence of normal PHA blasts led to amplification of HIV expression to levels detectable by FIA. In most instances, detection by FIA slightly preceded the appearance of reverse transcriptase. However, this kinetic difference was usually small under these assay conditions.

Chesebro et al., Virology 84, 222 (1978) have already found that detection of retrovirus-infected cells by infectivity assays is highly dependent on the rate of infectious virus release by individual cells. Cells releasing virus slowly are detected inefficiently, whereas cells releasing virus rapidly are readily detected. This situation appears also to hold true for cells infected with HIV. Previous studies of peripheral blood mononuclear cells from AIDS patients indicated that cells containing HIV gene sequences could be detected by in situ hybridization at a frequency of around 1 per $10^5$ cells in half of the patients. If the rate of virus production per cell were sufficiently high, such a frequency should be easily within the sensitivity of detection of these cells by FIA. The finding that FIA assays of cells taken directly from patients were consistently negative (i.e., less than one positive cell per $10^6$ cells) but became positive after in vitro co-cultivation, suggested that the rate of virus release was very low in patient peripheral blood cells but was elevated during in vitro cultivation. This conclusion agrees with estimates of very low HIV gene copy numbers observed by in situ hybridization of AIDS patient blood cells. Low virus expression is not usually seen in retroviral diseases, however, central nervous system "latent" infection by visna lentivirus is associated with the presence of a very low number of viral genes and low levels of viral protein and infectivity in infected cells. In this disease, the mechanism of regulation of expression is unknown, but low expression is believed to be important in long-term virus persistence in vivo. Interestingly, similar to the results with HIV, low expression of visna virus was reversed when cells were cultured in vitro.

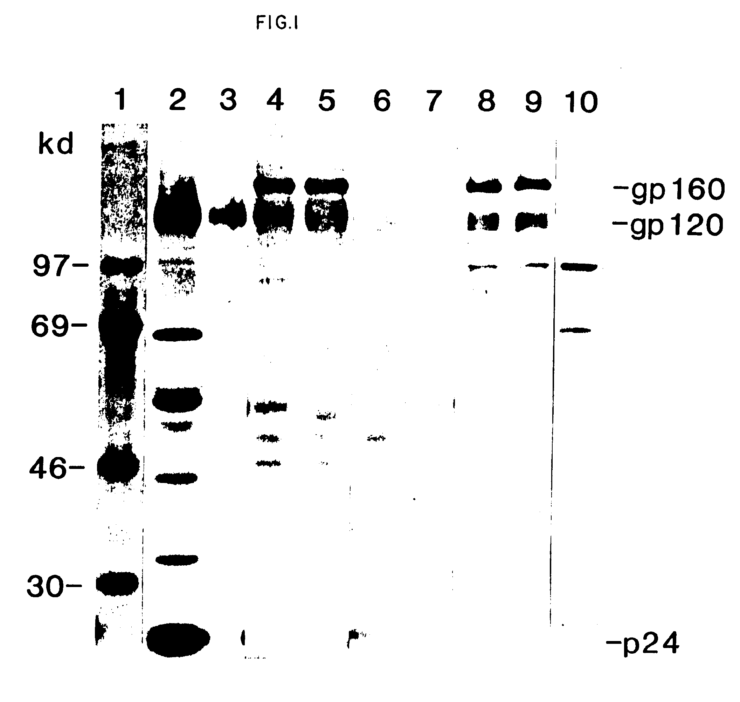

To obtain the results shown in Figure 1, HIV (LAV strain)-infected A3.01 cells were labelled with $^{35}$S-cysteine at 120 microcuries/ml and 2 x $10^6$ cells/ml for eight hours at 37°C in cysteine-free medium with dialyzed fetal calf serum. NIH/3T3 cells were infected with vaccinia expressing HIV gp$^{160}$ or vaccinia expressing influenza (HA) (18) at a multiplicity of 10, and twenty hours later cells were labelled with $^{35}$S-methionine at 100 microcuries/ml for two hours at 37°C in methionine-free medium.

After labelling, the cells were precleared and processed for immunoprecipitation using 9 x $10^5$ cell equivalents of lysate or 0.10 ml of NP40-lysed culture supernatant fluid incubated with 1 ml of hybridoma tissue culture supernatant or 3 microliters human AIDS patient serum. Then, 0.1 ml of a 10% suspension of Sepharose® protein A (Pharmacia) was added, incubated for thirty minutes with gentle rocking, and the beads were then washed three times with lysing buffer, eluted and analyzed by SDS-PAGE in 8% polyacrylamide gels followed by fluorography as described by Chesebro et al., Virology 112, 131 (1981).

In Lane 1, molecular weight markers are shown, Lane 2 shows human anti-HIV AIDS patient serum with supernatant of HIV-infected A3.01 cells. Lane 3 shows monoclonal antibody 902 with supernatant of HIV-infected A3.01 cells. Lane 4 shows human anti-HIV with lysate of HIV-infected A3.01 cells. Lane 5 shows monoclonal antibody 902 with lysate of HIV-infected A3.01 cells. Lane 6 shows human anti-HIV with lysate of uninfected A3.01 cells. Lane 7 shows monoclonal antibody 902 with lysate of uninfected A3.01 cells. Lane 8 shows monoclonal antibody 902 with lysate of Vaccinia-HIV gp160-infected NIH/3T3 cells. Lane 9 shows monoclonal antibody 907 with lysate of vaccinia-HIV gp 160-infected NIH/3T3 cells. Lane 10 shows monoclonal antibody 902 with lysate of vaccinia-influenza HA-infected NIH/3T3 cells. The locations of the HIV envelope proteins, gp160 and gp120, and the major HIV core protein, p24, are shown on the right.

Hybridomas producing monoclonal antibodies specific for HIV envelope proteins were obtained from spleen cells of (BIO.A (2R) X A.BY)F mice immunized with 1 x $10^7$ PFU of vaccinia virus expressing HIV gp$^{160}$ (1B) first in a tail scratch and three to five weeks later intraperitoneally. Three days after the second inoculation, spleen cells were fused with NS1 cells.

Supernatant fluids were screened by indirect immunofluorescence on methanol-fixed 8E5 cells which chronically express HIV envelope and gag proteins. Six positive clones were obtained from one mouse, and they appeared to represent at least two distinct clones (antibodies 902 and 907) based on differences in L chain migration in SDS-PAGE gels. All antibodies were of $IgG_1$ subclass, and in Lysing buffer, all bound to Sepharose protein A, but not to heat and formalin-fixed staph A (Cowan I strain). All antibodies were specific for HIV gp$^{120}$ based on reactivity in indirect immunofluorescence with methanol-fixed CV-1 cells infected with vaccinia expressing HIV gp160 or gp120. No reactivity was observed against CV-1 cells infected with vaccinia expressing influenza HA. Furthermore, these antibodies reacted in indirect immunofluorescence with live and methanol-fixed A3.01 cells and MOLT-4 cells infected with HIV (LAV strain), H9 cells infected with HIV (HTLV IIIB strain), and 8E5 cells, but were nonreactive with uninfected A3.01, MOLT4, and H9 cells, or with A3.01 cells or PHA blast cells infected with all other HIV isolates tested so far.

Figure 2A shows the foci of HIV (LAV strain)-infected HT4-6C cells detected by indirect immunofluorescence using monoclonal antibody 902, specific for HIV gp120. The field shown has a single focus of HIV-infected cells including some multi-nucleated giant cells and some mononuclear cells.

Figure 2B shows the foci of HIV (LAV)-infected HT4-6C cells showing one area of HIV-positive mononuclear cells and one giant cell with six nuclei.

Figures 2C and 2D show foci of HIV (5888 patient isolate) - infected HT4-6C cells detected by human AIDS patient serum and FITC-anti-human Ig. Both foci consist of a single multinucleated giant cell expressing HIV antigens. With wild-type HIV isolates, only rarely have a mononuclear HT4-6C cells expressing HIV been observed.

Figure 2E shows the focus of HIV(LAV)-infected HT4-6C cells detected using the FIA on a live cell monolayer. It should be noted that the multinucleated cells are not visible and appear to have been removed during the multiple washes of the FIA procedure.

The details of the focal immunoassay performed for these figures are as follows: $5 \times 10^4$ HT4-6C cells were seeded in 35 mm plastic tissue culture wells with 2.5 ml, RPMI 1640 plus 10% fetal calf serum. The following day, various numbers of HIV-infected A3.01 or PHA blast cells were added to the wells. Eighteen hours later, the suspended cells were removed, and HT4-6C cells were fed and allowed to grow for three more days. The medium was then removed, the attached cells were fixed five minutes with methanol, rinsed with PBS, and processed for indirect immunofluorescence. The wells were incubated for twenty minutes at room temperature with either 0.15 ml of hybridoma tissue culture fluid (antibody 902) or 0.15 ml of a 1/300 dilution of human AIDS patient serum selected for low background against uninfected HT4-6C cells. The cells were then rinsed with 2 ml PBS, incubated for thirty minutes with 0.15 ml of a 1/180 dilution of FITC-conjugated goat or sheep antibodies specific for either mouse or human immunoglobulins, rinsed again with PBS, and either examined immediately in an inverted fluorescence microscope as previously described by Sitbon et al., Virology 141, 110 (1985), or refixed for five minutes with methanol and stored in PBS with 0.002 M EDTA at 4°C for future examination. FIA on live infected cells was identical except that the original fixation with methanol was omitted.

Figure 2F shows the focus of HIV (LAV)-infected HT4-6C cells detected by FIA as in Figure 2A except that the second antibody was peroxidase-conjugated goat anti-mouse Ig, and peroxidase staining was developed using hydrogen peroxide and diaminobenzidine. The foci were visualized by low power light microscopy.

In Figure 3, the foci of HIV infection in HT4-6C cells were detected by FIA four days after infection with HIV-infected A3.01 cells after various treatments. Data shown were obtained using monoclonal 902 as the first antibody, but similar results were obtained with human anti-HIV serum followed by FITC-conjugated sheep anti-human immunoglobulin. A3.01 suspension cultures were used six days after infection with a 1/10 dilution of HIV (LAV strain). Infected cells were seeded directly, or were washed twice by centrifugation, resuspended at their original concentration ($3 \times 10^5$ cells/ml), and then treated with: 10,000 rad X-irradiation to prevent subsequent cell division, digestion for ten minutes with 0.02% trypsin in 2 mM EDAT with 0.15 ml NaCl to inactivate extracellular virus, or lysed by Dounce homogenization to kill cells directly. These samples as well as untreated control cells were then placed in HT4-6C cultures for analysis by FIA.

Figures 4A and 4B show the correlation of HIV-infected lymphoid cells detected by FIA and by indirect immunofluorescence at various times after infection. Lymphocyte blast cells were infected with an AIDS patient HIV isolate (5888P) three days after PHA stimulation. A3.01 cells were infected with HIV (LAV strain). Infected cells were cultured in vitro and assayed at various days by FIA on HT4-6C cells or by indirect immunofluorescence on methanol-fixed lymphoid cells using monoclonal antibody 902 and FITC-conjugated anti-mouse Ig for HIV LAV and human AIDS patient serum plus FITC-conjugated anti-human Ig for HIV-5888P.

In Figure 5, HIV (LAV strain) from infected A3.01 cell cultures was used to infect HT4-6C cell monolayers. Infection was accomplished by adding 0.5 ml of the dilutions shown to 3 ml culture medium without polybrene or DEAE-dextran. Twenty four hours later the medium was changed, and FIA was done on the fourth day after infection.

EXAMPLE I

The effects of drug therapy are monitored in patients suffering from AIDS by taking blood samples from the patients and testing for the presence of HIV-infected cells according to the above-described procedure. A decrease in the number of HIV-infected cells during the course of therapy is a positive indication of the efficacy of the drug against the disease.

EXAMPLE II

HIV expression can be quantified in asymptomatic HIV antibody-positive patients by testing for the presence of HIV in the blood of these patients according to the procedure of the present invention.

EXAMPLE III

HIV neutralizing activity in serum is measured by mixing serial dilutions of serum to be tested for neutralizing antibody with a dilution of HIV appropriate to give a countable number of foci in the FIA. After incubation of the mixture for 1 hour at 37°C the mixture is titered for residual HIV infectivity using the FIA described above. Elimination of infectious HIV by incubation with serum represents neutralization of HIV by serum antibodies.

## Claims

1. A method for assaying for human immunodeficiency virus, comprising seeding dishes with cells susceptible to infection and capable of growing on the dishes, adding a sample to be tested to the dishes, incubating the cells, exposing the cells to a first antibody and to a marker-conjugated antibody, reactive with the first antibody, and counting the number of foci of marker-positive cells per dish, **characterised in that** the seeded cells comprise a human carcinoma cell line, into which a CD4 gene encoding the HIV receptor molecule T4 has been transferred, and are sensitive to infection by wild-type HIV, the first antibody is reactive with an HIV protein and the number of foci, of marker-positive cells, is counted in order to determine the number of HIV infected cells in the dish.

2. The method of claim 1 wherein the antibody reactive with said first antibody is conjugated with a detectable marker.

3. The method of claim 2 wherein the marker is fluorescein isothiocyanate.

4. The method of claim 2 wherein the marker is horseradish peroxidase.

5. The method of claim 1 wherein the cells susceptible to HIV infection are HeLa cells which are T4-positive.

6. The method of claim 1 wherein the cells susceptible to HIV infection are brain cells which are T4-positive.

7. The method of claim 1 wherein the first antibody is a monoclonal antibody.

8. The method of claim 1 wherein the first antibody is reactive with the envelope protein of HIV.

9. The method of claim 1 wherein the time between seeding of the dishes and counting of the foci is from about two to about four days.

10. The method of claim 1 wherein the cells are fixed after the incubation step.

11. The method of claim 10 wherein the cells are fixed with a compound selected from the group consisting of methanol and formaldehyde.

12. A method for detecting HIV-infected cells in a sample, comprising seeding dishes with first cells susceptible to infection, seeding the dishes with second cells from the sample, incubating the cells, exposing the cells to a first antibody, exposing the cells to a marker-conjugated antibody, reactive with the first antibody, and counting the number of foci of marker-positive cells, **characterised in that** the first cells comprise a human carcinoma cell line, into which a CD4 gene encoding the HIV receptor molecule T4 has been transferred, and are susceptible to wild-type HIV infection, the first antibody is reactive with an HIV protein, and the number of foci of marker-positive cells is counted, to determine the number of HIV infected cells in the sample.

13. A method for monitoring the effects of drug therapy on an AIDS patient, comprising assaying sample cells from an AIDS patient according to the process of claim 12 to determine if the drug is affecting the cells of the AIDS patient.

14. A method for quantifying HIV expression in sample cells from HIV antibody-positive patients, comprising assaying said sample cells for HIV according to the method of claim 12.

15. A method as claimed in any of claims 1-14, wherein the seeded cells are capable of growing on the dishes as a monolayer of cells.

16. Target cells for use in a focal immunoassay for HIV, as claimed in any of claims 1-15, comprising a human carcinoma cell line into which a CD4 gene encoding the HIV receptor molecule T4 has been transferred.

17. Target cells, as claimed in claim 16, which are capable of growing on a plastic dish as a monolayer of cells.

18. A method for determining if cells have been infected with HIV comprising:
    seeding dishes with first cells which can be infected with HIV;
    seeding the dishes with sample cells;
    incubating said cells;
    exposing said cells to a first antibody reactive with HIV proteins;
    exposing said cells to a marker-conjugated antibody reactive with said first antibody;
    counting the marked foci of said cells; and
    determining the presence of marker-positive cells to indicated whether the sample cells have been infected with HIV; wherein the first cells comprise a human carcinoma cell line, into which a CD4 gene encoding the HIV receptor molecule T4 has been transferred, and are sensitive to wild-type HIV infection.

19. A method for assaying body fluids for HIV comprising using sample cells from said body fluid in the method according to claim 18.

20. The method of claim 19 wherein said body fluids are selected from the group consisting of blood, semen and urine.

## Patentansprüche

1. Ein Verfahren zur Prüfung auf Human Immunodeficiency Virus, umfassend Beimpfung von Schalen mit infektionsanfälligen und zum Wachsen auf den Schalen fähigen Zellen, Zusatz einer zu prüfenden Probe zu den besagten Schalen, Inkubation der Zellen, Aussetzung der besagten Zellen einem ersten Antikörper und einem mit dem ersten Antikörper reaktionsfähigen, mit Markierstoff konjugierten Antikörper und Zählen der Anzahl von Herden markierstoffpositiver Zellen je Schale, dadurch gekennzeichnet, daß die aufgeimpften Zellen eine Zellinie von menschlichem Krebs umfassen, in die ein das HIV-Rezeptormolekül T4 codierendes CD4-Gen transferiert wurde und daß sie für Infektion durch Wildtyp-HIV empfindlich sind, daß der erste Antikörper mit einem HIV-Protein reaktionsfähig ist und daß die Anzahl von Herden markierstoffpositiver Zellen gezählt wird, um die Anzahl von mit HIV infizierten Zellen in der Schale zu bestimmen.

2. Das Verfahren nach Anspruch 1, bei dem der mit dem besagten ersten Antikörper reaktionsfähige Antikörper mit einem nachweisbaren Markierstoff konjugiert ist.

3. Das Verfahren nach Anspruch 2, bei dem der Markierstoff Fluoresceinisothiocyanat ist.

4. Das Verfahren nach Anspruch 2, bei dem der Markierstoff Meerrettichperoxidase ist.

5. Das Verfahren nach Anspruch 1, bei dem die für HIV-Infektion anfälligen Zellen T4-positive HeLa-Zellen sind.

6. Das Verfahren nach Anspruch 1, bei dem die für HIV-Infektion anfälligen Zellen T4-positive Hirnzellen sind.

7. Das Verfahren nach Anspruch 1, bei dem der erste Antikörper ein monoklonaler Antikörper ist.

8. Das Verfahren nach Anspruch 1, bei dem der erste Antikörper mit dem Hüllprotein von HIV reaktionsfähig ist.

9. Das Verfahren nach Anspruch 1, bei dem die Zeitspanne zwischen dem Beimpfen der Schalen und dem Zählen der Herde von etwa zwei Tagen bis etwa vier Tagen beträgt.

10. Das Verfahren nach Anspruch 1, bei dem die Zellen nach der Inkubationsstufe fixiert werden.

11. Das Verfahren nach Anspruch 10, bei dem die Zellen mit einer aus der Methanol und Formaldehyd umfassenden Gruppe ausgewählten Verbindung fixiert werden.

12. Ein Verfahren zum Nachweisen von mit HIV infizierten Zellen in einer Probe, umfassend Beimpfung von Schalen mit ersten infektionsanfälligen Zellen, Beimpfung der Schalen mit zweiten Zellen aus der Probe, Inkubation der Zellen, Aussetzung der Zellen einem ersten Antikörper, Aussetzung der Zellen einem mit dem ersten Antikörper reaktionsfähigen Markierstoff konjugierten Antikörper und Zählen der Anzahl von Herden markierstoffpositiver Zellen, dadurch gekennzeichnet, daß die ersten Zellen eine Zellinie von menschlichem Krebs umfassen, in die ein das HIV-Rezeptormolekül T4 codierendes CD4-Gen transferiert wurde, und daß sie für Wildtyp-HIV-Infektion anfällig sind, daß der erste Antikörper mit einem HIV-Protein reaktionsfähig ist und daß die Anzahl von Herden markierstoffpositiver Zellen gezählt wird, um die Anzahl von mit HIV infizierten Zellen in der Probe zu bestimmen.

13. Ein Verfahren zur Überwachung der Auswirkungen von Drogentherapie auf einen AIDS-Patienten, umfassend Prüfung von von einem AIDS-Patienten stammenden Probezellen gemäß dem Verfahren nach Anspruch 12, um zu bestimmen, ob die Droge auf die Zellen des AIDS-Patienten eine Wirkung ausübt.

14. Ein Verfahren zu quantitativer HIV-Expression in Probezellen von HIV-Antikörper-positiven Patienten, umfassend Prüfung der besagten Probezellen auf HIV gemäß dem Verfahren nach Anspruch 12.

15. Ein Verfahren nach einem der Ansprüche 1-14, bei dem die aufgeimpften Zellen in der Lage sind, auf den Schalen in der Form einer einzigen Zellenschicht zu wachsen.

16. Zielzellen zum Gebrauch in einer herdorientierten Immunprüfung auf HIV nach einem der Ansprüche 1-15, umfassend eine Zellinie von menschlichem Krebs, in die ein das HIV-Rezeptormolekül T4 codierendes CD4-Gen transferiert wurde.

17. Zielzellen nach Anspruch 16, die in der Lage sind, auf einer Kunststoffschale in der Form einer einzigen Zellenschicht zu wachsen.

18. Ein Verfahren zur Bestimmung, ob Zellen mit HIV infiziert wurden, umfassend:
    Beimpfung von Schalen mit ersten Zellen, die mit HIV infiziert werden können;
    Beimpfung der Schalen mit Probezellen;
    Inkubation der besagten Zellen;
    Aussetzung der besagten Zellen einem ersten mit HIV-Proteinen reaktionsfähigen Antikörper;
    Aussetzung der besagten Zellen einem mit dem besagten ersten Antikörper reaktionsfähigen markierstoffkonjugierten Antikörper;
    Zählung der markierten Herde der besagten Zellen; und
    Bestimmung des Vorhandenseins von markierstoffpositiven Zellen, um festzustellen, ob die Probezellen mit HIV infiziert wurden; wobei die ersten Zellen eine Zellinie von menschlichem Krebs umfassen, in die ein das HIV-Rezeptormolekül T4 codierendes CD4-Gen transferiert wurde, und die für Wildtyp-HIV-Infektion anfällig sind.

19. Ein Verfahren zum Prüfen von Körperflüssigkeiten auf HIV, umfassend den Gebrauch von Probezellen aus der besagten Körperflüssigkeit gemäß dem Verfahren nach Anspruch 18.

20. Das Verfahren nach Anspruch 19, bei dem die besagten Körperflüssigkeiten aus der Blut, Samen und Harn umfassenden Gruppe ausgewählt sind.

## Revendications

1. Procédé pour quantifier le virus de l'immunodéficience humaine, comprenant l'ensemencement de boîtes avec des cellules susceptibles d'être infectées et capables de pousser sur les boîtes, l'addition d'un échantillon à tester sur les boîtes, l'incubation des cellules, la mise en contact des cellules avec un premier anticorps et avec un anticorps conjugué à un marqueur, réagissant avec le premier anticorps, et la détermination du nombre de foyers par boîte, des cellules donnant une réponse positive du marqueur, caractérisé en ce que les cellules ensemencées comprennent une lignée cellulaire de carcinome humain, dans laquelle un gène CD4 codant pour la molécule de récepteur T4 du VIH a été transféré, et en ce que les cellules ensemencées sont sensibles à une infection par le VIH de type sauvage, en ce que le premier anticorps réagit avec une protéine du VIH, et en ce que le nombre de foyers de cellules donnant une réponse positive du marqueur est déterminé de manière à déterminer le nombre de cellules infectées par le VIH, dans la boîte.

2. Procédé selon la revendication 1, selon lequel l'anticorps réagissant avec ledit premier anticorps est conjugué à un marqueur détectable.

3. Procédé selon la revendication 2, selon lequel le marqueur est l'isothiocyanate de fluorescéine.

4. Procédé selon la revendication 2, selon lequel le marqueur est la peroxydase de raifort.

5. Procédé selon la revendication 1, selon lequel les cellules susceptibles d'être infectées par le VIH sont des cellules HeLa qui donnent une réponse positive des T4.

6. Procédé selon la revendication 1, selon lequel les cellules susceptibles d'être infectées par le VIH sont des cellules de cerveau qui donnent une réponse positive des T4.

7. Procédé selon la revendication 1, selon lequel le premier anticorps est un anticorps monoclonal.

8. Procédé selon la revendication 1, selon lequel le premier anticorps réagit avec la protéine d'enveloppe du VIH.

9. Procédé selon la revendication 1, selon lequel la période entre l'ensemencement des boîtes et le décomptage des foyers est d'environ deux à environ quatre jours.

10. Procédé selon la revendication 1, selon lequel les cellules sont fixées après l'étape d'incubation.

11. Procédé selon la revendication 10, selon lequel les cellules sont fixées par un composé choisi dans le groupe consistant en le méthanol et le formaldéhyde.

12. Procédé pour quantifier, dans un échantillon, les cellules infectées par le VIH, comprenant l'ensemencement de boîtes par des premières cellules susceptibles d'être infectées, l'ensemencement des boîtes par des secondes cellules dudit échantillon, l'incubation des cellules, la mise en contact des cellules avec un premier anticorps, la mise en contact des cellules avec un anticorps conjugué à un marqueur, réagissant avec le premier anticorps, et la détermination du nombre de foyers de cellules donnant une réponse positive du marqueur, caractérisé en ce que les premières cellules comprennent une lignée cellulaire de carcinome humain, dans laquelle un gène CD4 codant pour la molécule de récepteur T4 du VIH a été transféré, et en ce qu'elles sont susceptibles d'être infectées par le VIH de type sauvage, en ce que le premier anticorps réagit avec une protéine du VIH, et en ce que le nombre de foyers de cellules donnant une réponse positive du marqueur est déterminé, pour déterminer le nombre de cellules infectées par le VIH, dans l'échantillon.

13. Procédé pour contrôler les effets d'une thérapie médicamenteuse chez un patient atteint du SIDA, comprenant la quantification des cellules d'un échantillon prélevé chez un patient atteint du SIDA, selon le procédé de la revendication 12, pour déterminer si le médicament affecte les cellules du patient atteint du SIDA.

14. Procédé pour quantifier l'expression du VIH dans des cellules d'un échantillon prélevé chez des patients donnant une réponse positive des anticorps du VIH, comprenant la quantification du VIH dans les cellules d'un échantillon, selon le procédé de la revendication 12.

15. Procédé selon l'une quelconque des revendications 1 à 14, selon lequel les cellules ensemencées sont capables de pousser sur des boîtes, en monocouches de cellules.

16. Cellules cibles destinées à être utilisées dans un immuno-essai focal du VIH, selon l'une quelconque des revendications 1 à 15, comprenant une lignée cellulaire de carcinome humain, dans laquelle un gène CD4 codant pour la molécule du récepteur T4 du VIH a été transféré.

17. Cellules cibles selon la revendication 16, capables de pousser sur une boîte plastique, en monocouches de cellules.

18. Procédé pour déterminer si les cellules ont été infectées par le VIH comprenant :
- l'ensemencement de boîtes avec des premières cellules qui peuvent être infectées par le VIH;
- l'ensemencement de boîtes avec des cellules d'un échantillon;
- incubation desdites cellules;
- la mise en contact desdites cellules avec un premier anticorps réagissant avec les protéines du VIH;
- la mise en contact desdites cellules avec un anticorps conjugué à un marqueur réagissant avec ledit premier anticorps;
- décomptage des foyers marqués desdites cellules, et
- détermination de la présence de cellules donnant une réponse positive du marqueur pour indiquer si les cellules de l'échantillon ont été infectées par le VIH; procédé selon lequel les premières cellules comprennent une lignée cellulaire de carcinome humain, dans laquelle un gène CD4 codant pour la molécule du récepteur T4 du VIH a été transféré, et en ce qu'elles sont sensibles à une infection par le VIH de type sauvage.

19. Procédé pour quantifier le VIH dans des fluides corporels, comprenant l'utilisation de cellules d'un échantillon prélevé sur ledit fluide corporel dans un procédé selon la revendication 18.

20. Procédé selon la revendication 19, selon lequel lesdits fluides corporels sont choisis dans le groupe consistant en le sang, le sperme et l'urine.

# FIG.I

FIG.I

1  2  3  4  5  6  7  8  9  10

kd

97-

69-

46-

30-

-gp160
-gp120

-p24

EP 0 406 303 B1

FIG.2A
FIG.2B
FIG.2C
FIG.2D
FIG.2E
FIG.2F

FIG.3

EP 0 406 303 B1

FIG.4A

FIG.4B

FIG.5